(19) Europäisches Patentamt
European Patent Office
Office européen des brevets

(11) Veröffentlichungsnummer: **0 270 962**
**A2**

(12) # EUROPÄISCHE PATENTANMELDUNG

(21) Anmeldenummer: **87117650.9**

(22) Anmeldetag: **28.11.87**

(51) Int. Cl.⁴: **C09B 57/00** , C07D 519/00 , //C08K5/34,C09B57/04,(C07D519/00,487:00,487:00)

(30) Priorität: **10.12.86 DE 3642104**

(43) Veröffentlichungstag der Anmeldung: **15.06.88 Patentblatt 88/24**

(84) Benannte Vertragsstaaten: **CH DE FR GB LI**

(71) Anmelder: **BAYER AG**
**Konzernverwaltung RP Patentabteilung**
**D-5090 Leverkusen 1 Bayerwerk(DE)**

(72) Erfinder: **Meinhard, Rolf, Dr.**
**Berta-von-Suttner-Strasse 24**
**D-5090 Leverkusen(DE)**

(54) **Heterocyclische Verbindungen, Verfahren zu ihrer Herstellung sowie ihre Verwendung.**

(57) Heterocyclische Verbindungen der Formel

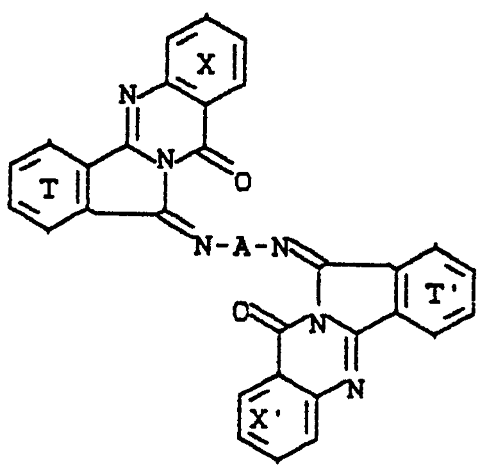

(I)

in der die mit X, X', T und T' bezeichneten Ringe substituiert sein können und durch gegebenenfalls substituierte anellierte Benzoringe zu Naphthalinsystemen erweitert sein können und A für einen zweiwertigen organischen Rest steht
sowie die Verwendung dieser Verbindungen zum Pigmentieren von organischem Material.

EP 0 270 962 A2

BAYER AKTIENGESELLSCHAFT 5090 Leverkusen, Bayerwerk
Konzernverwaltung RP
Patentabteilung PG/by-c

# Heterocyclische Verbindungen, Verfahren zu ihrer Herstellung sowie ihre Verwendung

Die Erfindung betrifft heterocyclische Verbindungen der Formel

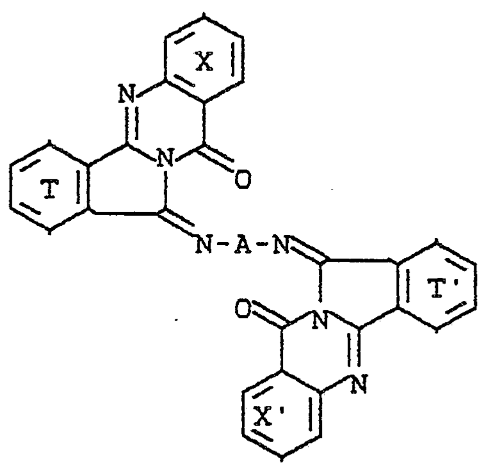

(I)

Verfahren zu ihrer Herstellung und ihre Verwendung.

In der Formel (I) können die mit X, X', T und T' bezeichneten Ringe substituiert sein, beispielsweise durch jeweils 1, 2, 3 oder 4 Substituenten aus der Reihe

Halogen, insbesondere Chlor und Brom; $C_1$-$C_6$-Alkyl, insbesondere Methyl; gegebenenfalls substituiertes Aryl, insbesondere Phenyl, das z.B. durch Halogen wie Chlor und Brom, $C_1$-$C_6$-Alkyl wie Methyl, Acylamino wie Acetylamino oder Benzoylamino, Nitro, Carboxy oder Carbamoyl substituiert sein kann; $C_1$-$C_6$-Alkoxy, insbesondere Methoxy; Acylamino insbesondere Acetylamino und Benzoylamino; Carboxy; Nitro oder Carbamoyl. Die Ringe X, X', T und T' können darüber hinaus durch eventuell substituierte anellierte Benzoringe zu Naphthalinsystemen erweitert sein. Als Substituenten in den Benzoringen kommen z.B. Halogen wie Chlor und Brom, $C_1$-$C_6$-Alkyl wie Methyl, $C_1$-$C_6$-Alkoxy, insbesondere Methoxy, Acylamino wie Acetylamino oder Benzoylamino, Nitro, Carboxy oder Carbamoyl in Frage.

A in Formel (I) steht für einen zweiwertigen organischen Rest.

Vorzugsweise wird A so gewählt, daß die Verbindung (I) Pigmenteigenschaften aufweist.

Weiterhin bevorzugt bezeichnet A einen Rest, der mindestens einen Benzol-, Naphthalin- oder Diphenylring enthält, wobei diese Ringe substituiert sein können.

Beispiele für A sind Reste der Formeln

$$-Ar- \quad \text{und} \quad -Ar-G-Ar^1-$$

wobei

Ar, $Ar^1$ für Phenylen, Naphthylen oder Biphenylylen stehen, die substituiert sein können und

G ein Brückenglied bezeichnet, vorzugsweise -S-, -O-, $-SO_2-$, -CO-, -NHCO-, -NHCONH-, -CONHCO-, -CONHNHCO-, -NHCO-Ar-CONH- mit der oben angegebenen Definition von Ar, -N=N-, -CH=CH-, -CH=N-, -NHCOCONH-, -CONH-Ar-CONH- mit der oben angegebenen Definition von Ar.

Weiterhin können die Reste auch heterocyclische Struktureinheiten enthalten.

Speziellere Beispiele für A sind:

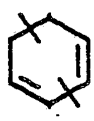 , 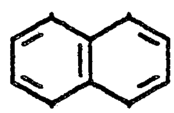 , 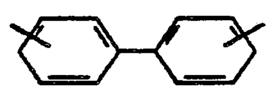 , 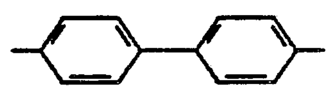 ,

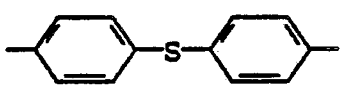 ,

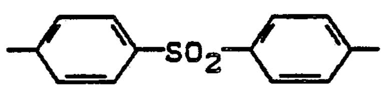 , 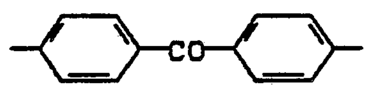 ,

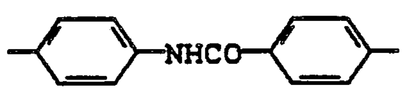 , 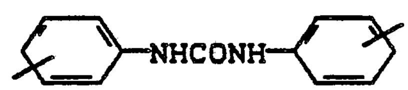 ,

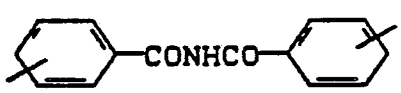 , 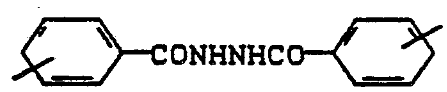 ,

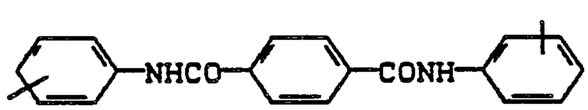 ,

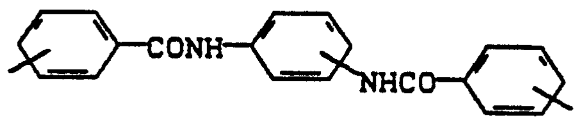 , 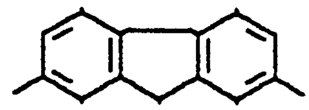 ,

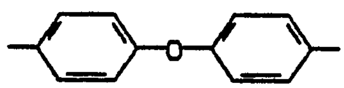 ,

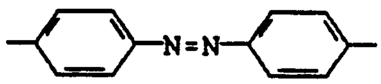 , 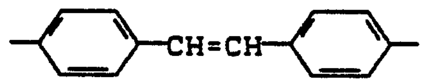 ,

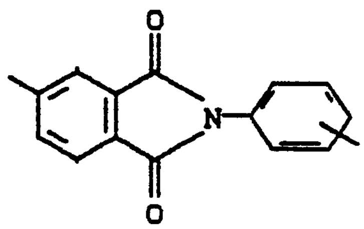 , 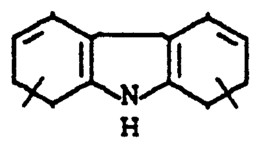 ,

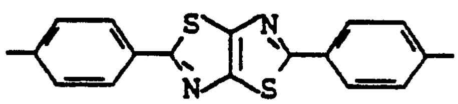 , 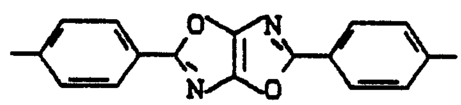 ,

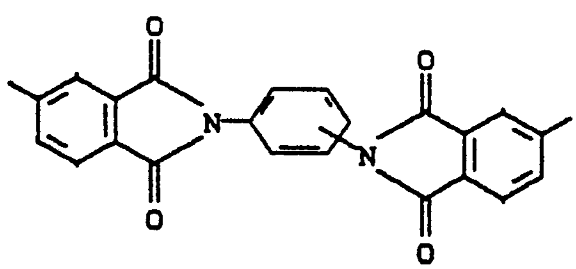 , 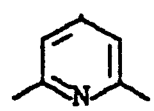

Als Substituenten für die Benzol-, Naphthalin- oder Diphenylringe der Reste A seien beispielsweise Halogen, insbesondere Chlor und Brom, Alkyl, insbesondere $C_1$-$C_6$-Alkyl, z.B. Methyl, Nitro, Alkoxy, insbesondere $C_1$-$C_6$-Alkoxy, z.B. Methoxy, Acylamino, insbesondere $C_1$-$C_6$-Alkylcarbonylamino, z.B. Acetylamino, oder Benzoylamino, Cyan, Carboxy, $C_1$-$C_6$-Alkoxycarbonyl, z.B. Methoxycarbonyl, genannt.

Aufgrund der bevorzugten Herstellungsweise der Verbindungen der Formel (I) kann man A auch als Reste von Diaminoverbindungen ansehen.

Bevorzugt sind symmetrische Verbindungen der Formel

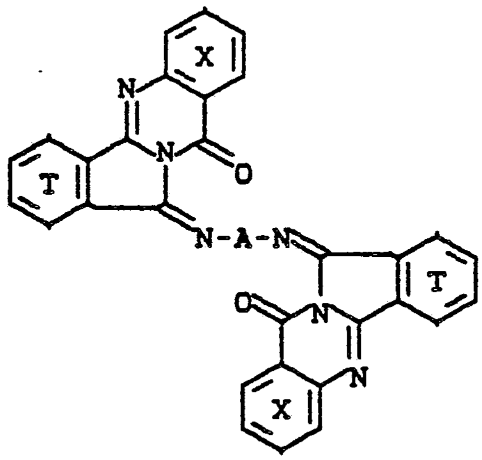

(II)

mit den obengenannten Bedeutungen für X, T und A.

Weiterhin bevorzugt sind Verbindungen der Formeln (I) und (II), in denen die durch X, X' und/oder T, T' markierten Ringe Benzolringe darstellen, gegebenenfalls substituiert durch Halogen, Methyl, Methoxy, Acetylamino, Benzoylamino, Carboxy, Carbamoyl oder Nitro. Weiterhin bevorzugt sind die Verbindungen der Formeln (I) und (II), bei denen A für einen gegebenenfalls durch Halogen, Methyl, Methoxy, Carbonyl, Methoxycarbonyl oder Nitro substituierten Phenylen-, Naphthylen- oder 4,4'-Biphenylylenrest steht.

Die neuen Verbindungen der Formel (I) werden vorzugsweise erhalten durch Umsetzung von Verbindungen der Formeln (III) und (IIIa):

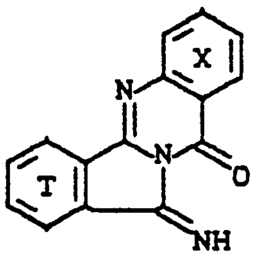 (III) 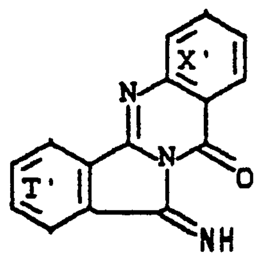 (IIIa)

mit einem Diamin der Formel

$$H_2N\text{-}A\text{-}NH_2 \qquad (IV).$$

In den Formeln (III) bzw. (IIIa) und (IV) haben X, X', T, T' und A die zu Formel (I) angegebenen Bedeutungen.

Dazu arbeitet man zweckmäßig in Wasser oder im organischen Lösungsmittel bei sauren Bedingungen und erhöhter Temperatur, bevorzugt bei 50 bis 180$^0$C. Geeignete Lösungsmittel sind Alkohole wie Methanol, Ethanol, Amylalkohol oder Glykolmonoalkylether; Aromaten wie Chlorbenzol, Nitrobenzol, Toluol; amidische Lösungsmittel wie Formamid, Dimethylformamid, N-Methylpyrrolidon; oder Säuren wie Ameisensäure oder Essigsäure.

Geeignete Säuren zur Herstellung der sauren Bedingungen sind anorganische wie Salzsäure, Schwefelsäure oder Phosphorsäure bzw. organische wie Ameisensäure, Essigsäure, Chloressigsäure, Dichloressigsäure, Oxalsäure, Benzosulfonsäure oder p-Toluolsulfonsäure.

Anstelle der Zwischenprodukte der Formeln (III) und (IIIa) können auch deren Acylierungsprodukte der Formeln (V) und (Va) eingesetzt werden:

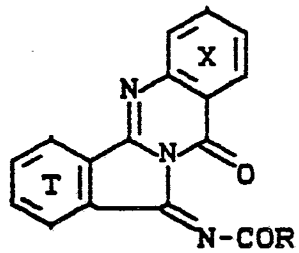 (V) 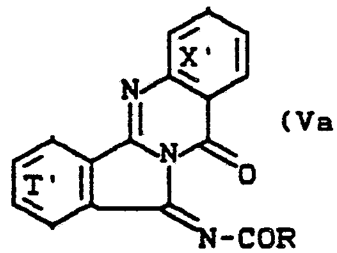 (Va)

In den Formeln (V) und (Va) haben T, T', X und X' die zu Formel (I) angegebenen Bedeutungen; R bezeichnet vorzugsweise Alkyl, vorzugsweise $C_1$-$C_6$-Alkyl, insbesondere Methyl oder Aryl, insbesondere Phenyl.

Die Zwischenprodukte der Formeln (III) bzw. (IIIa) und (V) bzw. (Va) sind literaturbekannt bzw. nach analogen Verfahren herstellbar.

Die Verbindungen der Formel (III) und (IIIa) bzw. (V) und (Va) können in beliebigem Verhältnis zueinander umgesetzt werden. Vorzugsweise handelt es sich um gleiche Verbindungen, deren Umsetzungen zu den symmetrischen Verbindungen der Formel (II) führen.

Die Verbindungen der Formel (I) fallen in einer für die Pigmentanwendung geeigneten Form an oder können durch an sich bekannte Nachbehandlungsverfahren in die geeignete Form überführt werden, z.B. durch Lösen oder Quellen in starken anorganischen Säuren wie Schwefelsäure und Austragen auf Eis. Die Feinverteilung kann

auch durch Mahlen mit oder ohne Mahlhilfsstoffen wie anorganische Salze oder Sand, gegebenenfalls in Anwesenheit von Lösungsmitteln wie Toluol, Xylol, Dichlorbenzol oder N-Methylpyrrolidon erzielt werden. Farbstärke und Transparenz des Pigments können durch Variation der Nachbehandlung beeinflußt werden.

Die Farbmittel der Formel (I) eignen sich aufgrund ihrer Licht- und Migrationsechtheit für die verschiedensten Pigmentapplikationen. Sie können zur Herstellung von sehr echt pigmentierten Systemen, wie Mischung mit anderen Stoffen, Zubereitungen, Anstrichmitteln, Druckfarben, gefärbtem Papier und gefärbten makromolekularen Stoffen verwendet werden. Unter Mischung mit anderen Stoffen können z.B. solche mit anorganischen Weißpigmenten wie Titandioxid (Rutil) oder mit Zement verstanden werden. Zubereitungen sind z.B. Flushpasten mit organischen Flüssigkeiten oder Teige und Feinteige mit Wasser, Dispergiermitteln und gegebenenfalls Konservierungsmitteln. Die Bezeichnung Anstrichmittel steht z.B. für physikalisch oder oxidativ trocknende Lacke, Einbrennlacke, Reaktionslacke, Zweikomponentenlacke, Dispersionsfarben für wetterfeste Überzüge und Leimfarben. Unter Druckfarben sind solche für den Papier-, Textil- und Blechdruck zu verstehen.

Die makromolekularen Stoffe können natürlichen Ursprungs sein, wie Kautschuk, durch chemische Modifikation erhalten werden wie Acetylcellulose, Cellulosebutyrat

oder Viskose oder synthetisch erzeugt werden wie Polymerisate, Polyadditionsprodukte und Polykondensate. Genannt seien plastische Massen wie Polyvinylchlorid, Polyvinylacetat, Polyvinylpropionat, Polyolefine, z.B. Polyethylen oder Polyamide, Superpolyamide, Polymerisate und Mischpolymerisate aus Acrylestern, Methacrylestern, Acrylnitril, Acrylamid, Butadien, Styrol sowie Polyurethane und Polycarbonate. Die mit den beanspruchten Produkten pigmentierten Stoffe können in beliebiger Form vorliegen.

Die Pigmente der Formel (I) sind weiterhin ausgezeichnet wasserecht, ölecht, säureecht, kalkecht, alkaliecht, lösungsmittelecht, überlackierecht, überspritzecht, sublimierecht, hitzebeständig, vulkanisierbeständig, sehr ergiebig und in plastischen Massen gut verteilbar.

Beispiel 1

In 100 ml Eisessig werden 15 g 12-Imino-isoindolo-(1,2-b)chinazolin-10(12H)-on (Formel III, unsubstituiert) mit 3 g p-Phenylendiamin 1 Stunde am Rückflußkühler gekocht. Anschließend wird heiß isoliert, mit Eisessig und Wasser gewaschen und getrocknet. Man erhält 12 g (75 %) einer gelben Verbindung der Formel

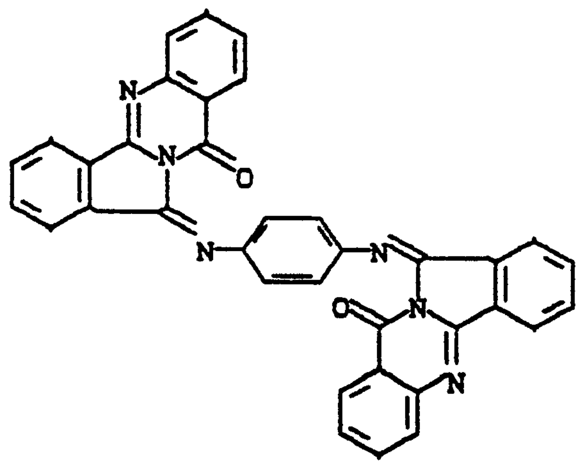

Schmelzpunkt: >300° C
Massenspektrum m/e (rel. Intensität): 568 $M^+$ (100 %), 338 (10), 230 (20), 102 (20).
IR-Spektrum ($cm^{-1}$): 1700, 1620, 1590, 1460, 1320, 1295, 940, 840, 760.

Analog Beispiel 1 erhält man bei Einsatz der in der folgenden Tabelle angegebenen Diamine anstelle von p-Phenylendiamin entsprechende Dimerisierungsprodukte mit den angegebenen Farbtönen.

| Beispiel | Diamin | Farbton |
|---|---|---|
| 2 | 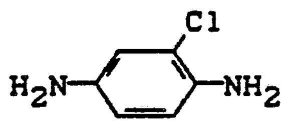 | rotstichig-gelb |
| 3 | 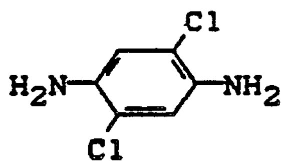 | rotstichig-gelb |
| 4 | 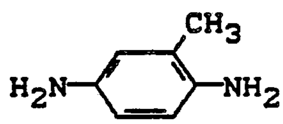 | gelb |
| 5 | 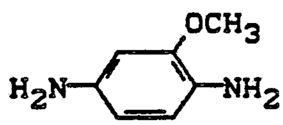 | orange |
| 6 | 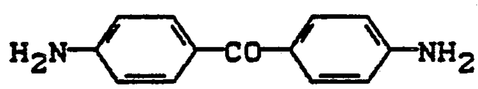 | grünstichig-gelb |
| 7 | 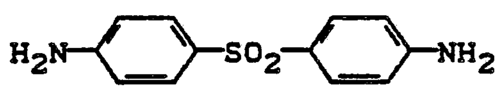 | grünstichig-gelb |
| 8 | 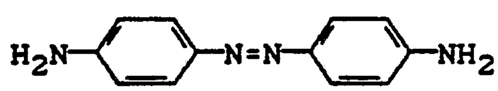 | braun |
| 9 | 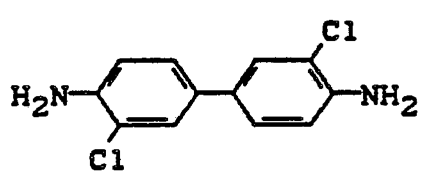 | gelb |

| Beispiel | Diamin | Farbton |
|---|---|---|
| 10 | 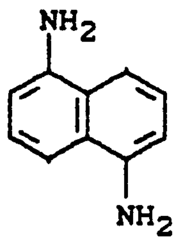 | orange |
| 11 | 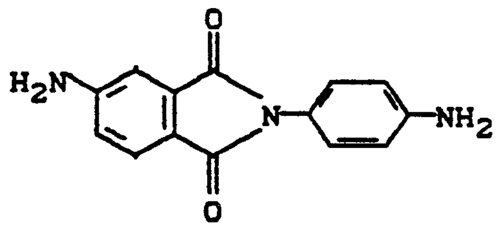 | rotstichig-gelb |
| 12 | 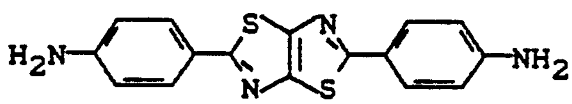 | orange |
| 13 | 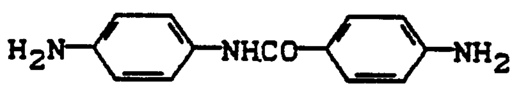 | braungelb |
| 14 | 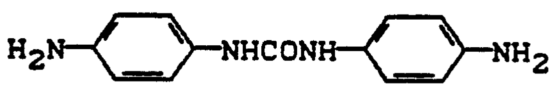 | rotstichig-gelb |
| 15 | 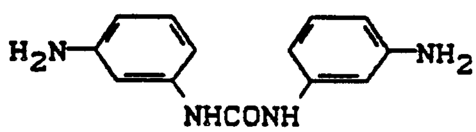 | rotstichig-gelb |
| 16 | 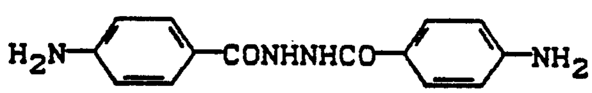 | gelb |
| 17 | 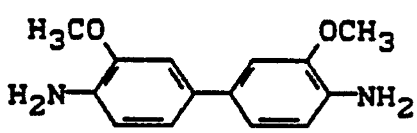 | rot |

Analog Beispiel 1 erhält man mit substituierten Isoindolzwischenprodukten und den in der Tabelle angegebenen Diaminen die entsprechenden dimeren Verbindungen mit den angegebenen Farbtönen.

| Beispiel | Isoindol | Diamin | Farbton |
|---|---|---|---|
| 18 | 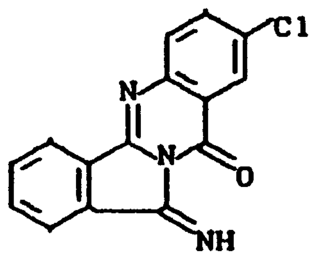 | 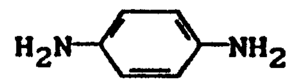 | gelb |
| 19 | 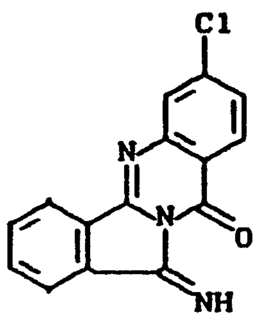 | 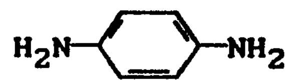 | gelb |
| 20 | 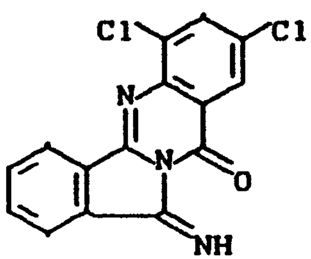 | 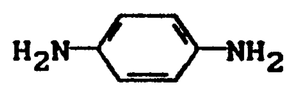 | gelb |

| Beispiel | Isoindol | Diamin | Farbton |
|---|---|---|---|
| 21 | 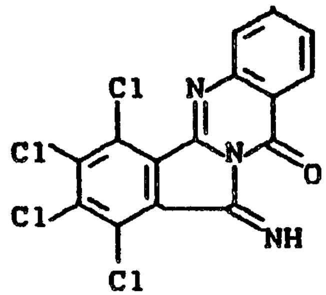 | 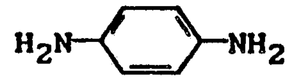 | gelb |
| 22 | 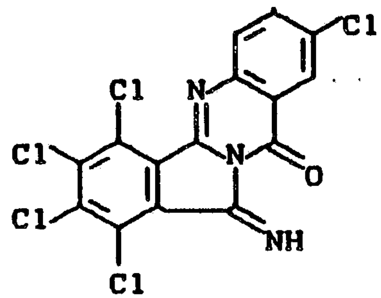 | 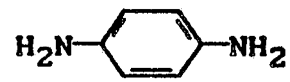 | gelb |
| 23 | 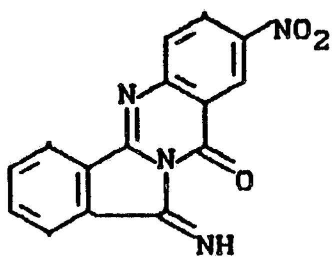 | 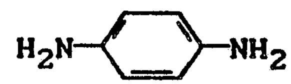 | rotstichig-gelb |

| Beispiel | Isoindol | Diamin | Farbton |
|---|---|---|---|
| 24 | 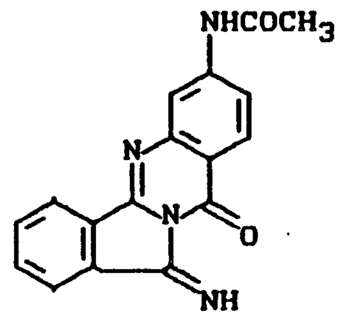 | 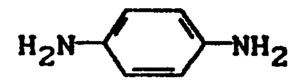 | rotstichig-gelb |
| 25 | 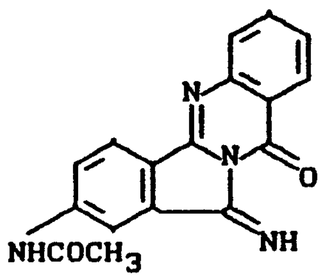 | 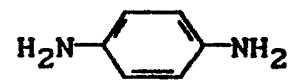 | orange |
| 26 | 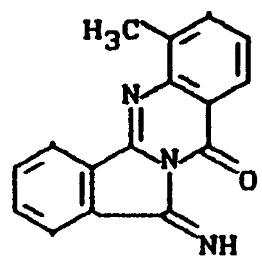 | 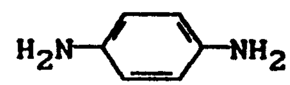 | orange |

| Beispiel | Isoindol | Diamin | Farbton |
|---|---|---|---|
| 27 | 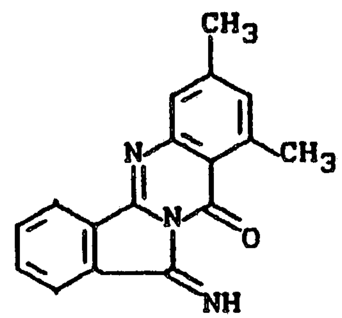 | 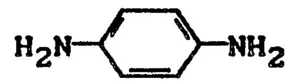 | orange |
| 28 | 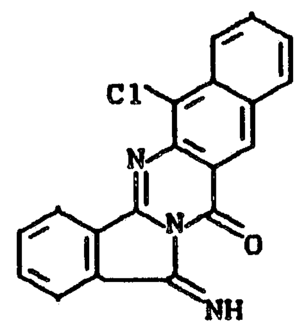 | 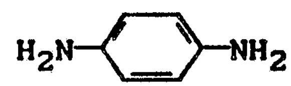 | orange |
| 29 | 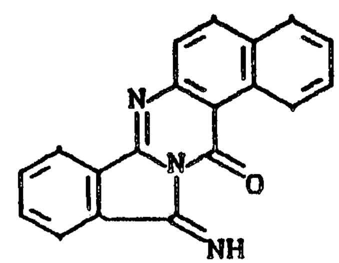 | 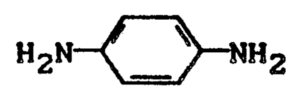 | braun |

| Beispiel | Isoindol | Diamin | Farbton |
|---|---|---|---|
| 30 | 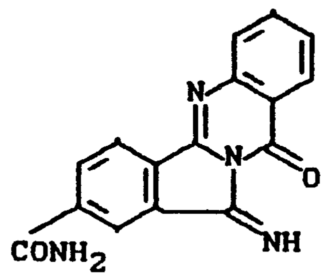 | 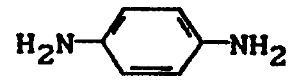 | gelb |
| 31 | 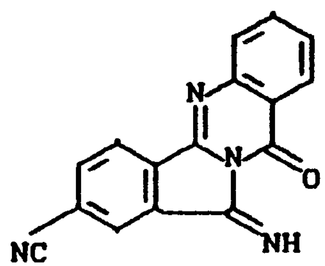 | 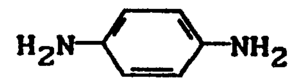 | gelb |
| 32 | 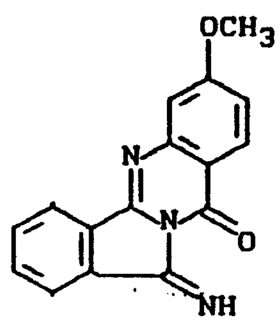 | 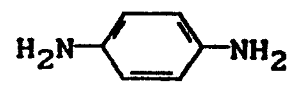 | rot |

| Beispiel | Isoindol | Diamin | Farbton |
|---|---|---|---|
| 33 | 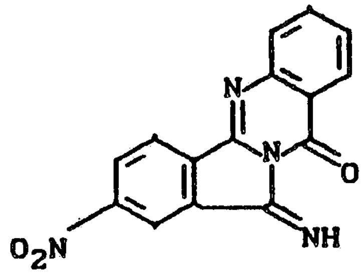 | 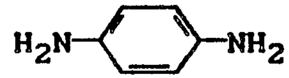 | gelb |
| 34 | 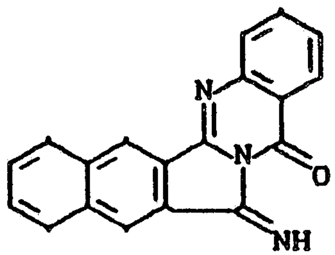 | 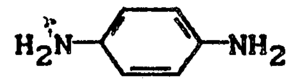 | orange |
| 35 | 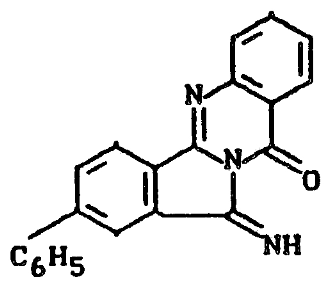 | 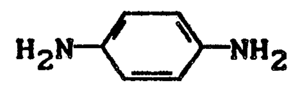 | orange |

| Beispiel | Isoindol | Diamin | Farbton |
| --- | --- | --- | --- |
| 36 | 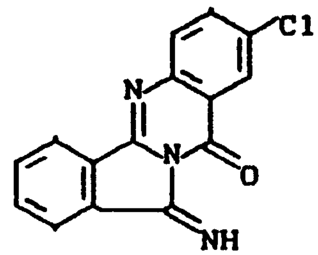 | 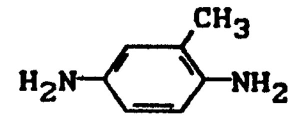 | orange |
| 37 | 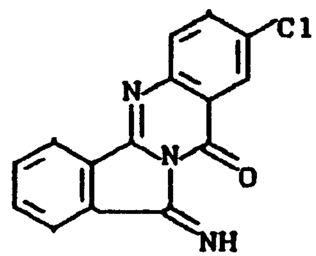 | 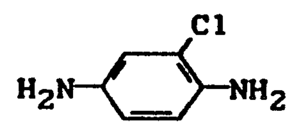 | gelb |
| 38 | 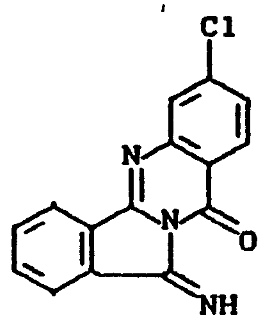 | 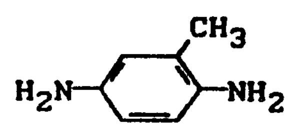 | gelb |

| Beispiel | Isoindol | Diamin | Farbton |
|---|---|---|---|
| 39 | 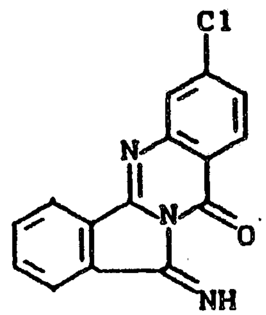 | 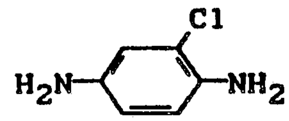 | gelb |
| 40 | 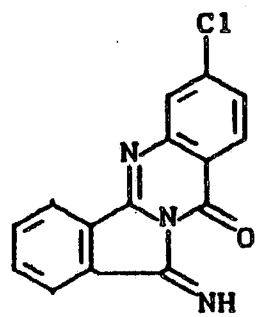 | 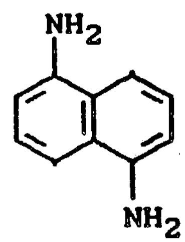 | orange |
| 41 | 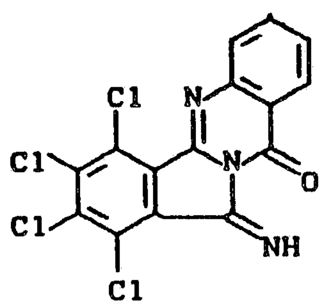 | 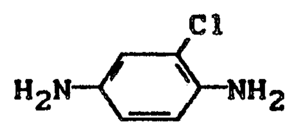 | gelb |

| Beispiel | Isoindol | Diamin | Farbton |
|---|---|---|---|
| 42 | 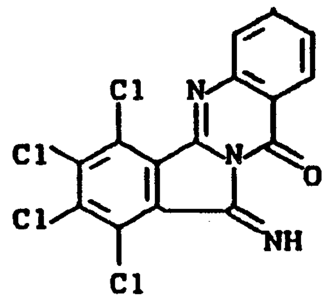 | 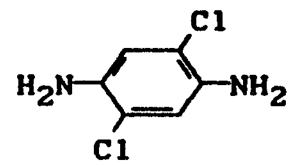 | gelb |
| 43 | 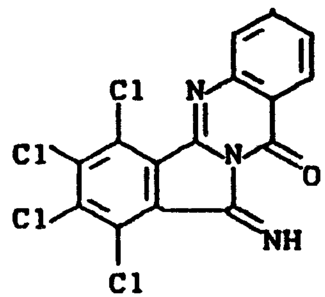 | 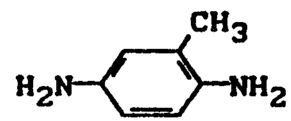 | orange |
| 44 | 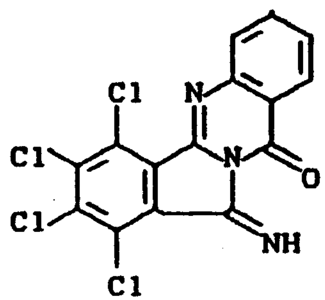 | 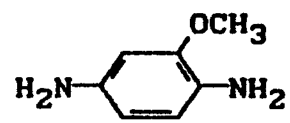 | orange |

| Beispiel | Isoindol | Diamin | Farbton |
|---|---|---|---|
| 45 | 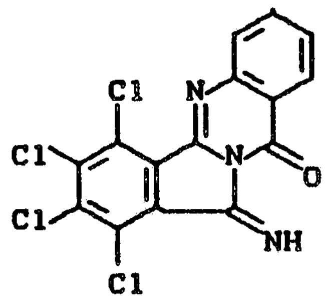 | 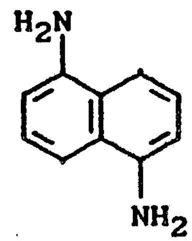 | orange |
| 46 | 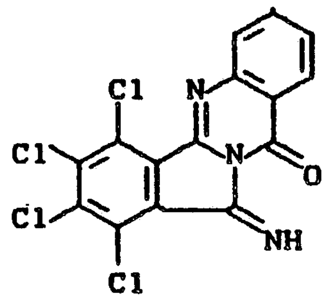 | 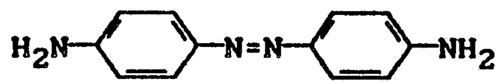 | orange |
| 47 | 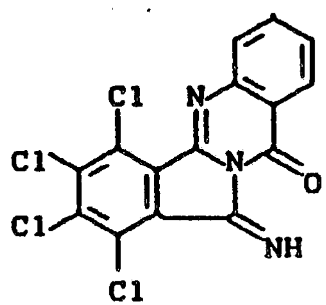 | 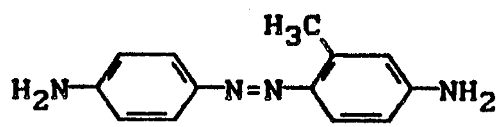 | orange |

Beispiel 48

In 100 ml Eisessig werden 7,5 g Verbindung (VI) und 8,6 g Verbindung (VII) mit 3 g p-Phenylendiamin 3 Stunden am Rückfluß gekocht. Anschließend wird heiß

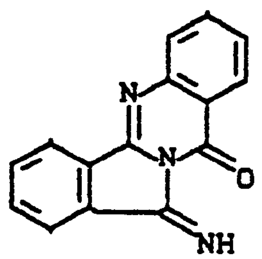

(VI)

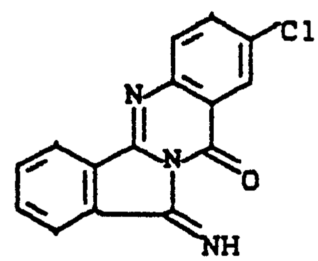

(VII)

isoliert, mit Eisessig und Wasser gewaschen und getrocknet.

Man erhält 14,5 g einer gelben Verbindung.
Elementaranalyse: C: 71,5 %, H: 3,3 %, N: 14,0 %, Cl: 6,0 %

Beispiel 49 (Anwendungsbeispiel)

8 g feingemahlenes Pigment gemäß Beispiel 1 werden in 92 g eines Einbrennlackes folgender Zusammensetzung dispergiert:

33 % Alkydharz
15 % Melaminharz
5 % Glykolmonomethylether
34 % Xylol
13 % Butanol

Als Alkydharze kommen Produkte auf Basis synthetischer und pflanzlicher Fettsäure wie Kokosöl, Rizinusöl, Rizinenöl, Leinöl u.a. in Frage. Anstelle von Melaminharzen können Harnstoffharze verwendet werden.

Nach erfolgter Dispergierung wird der pigmentierte Lack auf Papier-, Glas-, Kunststoff- oder Metall-Folien aufgetragen und 30 Minuten bei 130°C eingebrannt. Die Lackierungen besitzen sehr gute Licht- und Wetterbeständigkeit sowie gute Überlackierechtheit.

Beispiel 50 (Anwendungsbeispiel)

0,2 g Pigment nach Beispiel 1 werden mit 100 g Polyethylen-, Polypropylen- oder Polystyrolgranulat gemischt. Die Mischung kann entweder bei 220 bis 280°C direkt in einer Spritzgußmaschine verspritzt oder in einer Strangpresse zu gefärbten Stäben bzw. auf dem Mischwalzwerk zu gefärbten Fellen verarbeitet werden. Die Stäbe bzw. Felle werden gegebenenfalls granuliert und in einer Spritzgußmaschine verspritzt.

Die gelben Formlinge besitzen sehr gute Licht- und Migrationsechtheit. In ähnlicher Weise können bei 280

bis 300° C, gegebenenfalls unter Stickstoffatmosphäre, synthetische Polyamide aus Caprolactam oder Adipinsäure und Hexamethylendiamin oder die Kondensate aus Terephthalsäure und Ethylenglykol gefärbt werden.

Beispiel 51 (Anwendungsbeispiel)

Mit einer Druckfarbe, hergestellt durch Anreiben von 35 g Pigment nach Beispiel 1 und 65 g Leinöl und Zugabe von 1 g Siccativ (Co-Naphthenat, 50 %ig in Testbenzin) werden gelbe Offset-Drucke hoher Brillanz und Farbstärke und sehr gute Licht- und Lackierechtheiten erhalten. Verwendung dieser Druckfarbe in Buch-, Licht-, Stein- oder Stahlstichdruck führt zu gelben Drucken ähnlicher Echtheiten. Verwendet man das Pigment zur Färbung von Blechdrucken oder niedrigviskosen Tiefdrucken oder Drucktinten, erhält man Drucke ähnlicher Echtheiten.

Patentansprüche

1. Heterocyclische Verbindungen der Formel

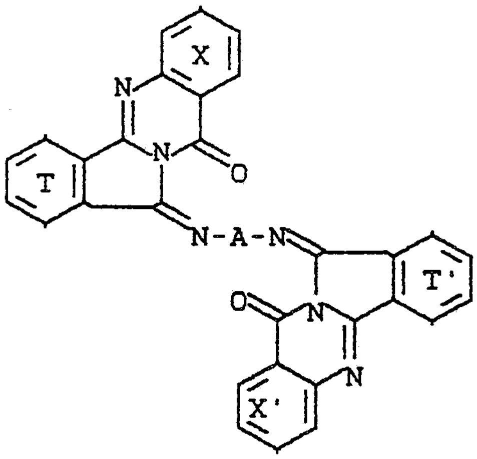

(I)

in der die mit X, X', T und T' bezeichneten Ringe substituiert sein können und durch gegebenenfalls substituierte anellierte Benzoringe zu Naphthalinsystemen erweitert sein können und A für einen zweiwertigen organischen Rest steht.

2. Heterocyclische Verbindungen gemäß Anspruch 1, wobei die mit X, X', T und T' bezeichneten Ringe durch jeweils 1, 2, 3 oder 4 Substituenten aus der Reihe Halogen, $C_1$-$C_6$-Alkyl, gegebenenfalls substituiertes Aryl, $C_1$-$C_6$-Alkoxy, Acylamino, Carboxy, Nitro, Carbamoyl substituiert sein können und durch anellierte Benzoringe, die durch Halogen, $C_1$-$C_6$-Alkyl, $C_1$-$C_6$-Alkoxy, Acylamino, Carboxy, Nitro und Carbamoyl substituiert sein können, zu Naphthalinsystemen erweitert sein können.

3. Heterocyclische Verbindungen gemäß den Ansprüchen 1 und 2, bei denen die mit X, X' und/oder T, T' markierten Ringe Benzolringe darstellen, die gegebenenfalls durch Methyl, Methoxy, Acetylamino, Benzoylamino, Carboxy, Carbamoyl oder Nitro substituiert sind.

4. Heterocyclische Verbindungen gemäß den Ansprüchen 1 bis 3, bei denen A so gewählt wird, daß die Verbindungen Pigmenteigenschaften aufweisen.

5. Heterocyclische Verbindungen gemäß den Ansprüchen 1 bis 4, bei denen A einen Rest bezeichnet, der mindestens einen Benzol-, Naphthalin- oder Diphenylring enthält.

6. Verbindungen gemäß den Ansprüchen 1 bis 5, bei denen A für

$$-Ar- \quad \text{oder} \quad -Ar-G-Ar^1-$$

steht, wobei

| | |
|---|---|
| Ar, $Ar^1$ | Phenylen, Naphthylen oder Biphenylylen, die substituiert sein können, bezeichnen und |
| G | für ein Brückenglied steht. |

7. Heterocyclische Verbindungen gemäß Anspruch 6, bei denen

G -S-, -O-, $-SO_2-$, -CO-, -NHCO-, -NHCONH-, -CONHCO-, -CONHNHCO-, -NHCO-Ar-CONH-, -N=N-, -CH=CH-, -CH=N-, -NHCOCONH-, -CONH-Ar-CONH- bezeichnet.

8. Verbindungen gemäß den Ansprüchen 1 bis 7, bei denen A einen gegebenenfalls durch Halogen, Methyl, Methoxy, Carboxy, Methoxycarbonyl oder Nitro substituierten Phenylen-, Naphthylen- oder 4,4'-Biphenylylen-Rest bezeichnet.

9. Symmetrische Verbindungen der Formel

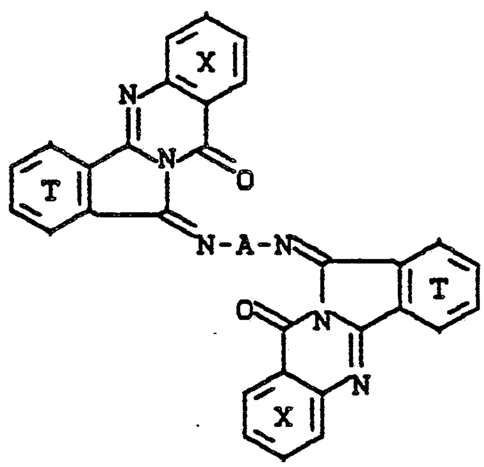

(II)

gemäß den Ansprüchen 1 bis 8.

10. Verfahren zum Pigmentieren von organischem Material, dadurch gekennzeichnet, daß man heterocyclische Verbindungen gemäß den Ansprüchen 1 bis 9 einsetzt.